# EUROPÄISCHE PATENTSCHRIFT

(11) **EP 3 463 124 B1**
(45) Veröffentlichungstag und Bekanntmachung des Hinweises auf die Patenterteilung: **20.01.2021**
(21) Anmeldenummer: 17722020.9
(22) Anmeldetag: 08.05.2017
(51) Int. Cl.: A61B 17/34, G01R 33/28, A61B 90/11, A61B 34/10

(54) **VERFAHREN ZUR PLANUNG EINER INTRAKORPORALEN VERORTUNG EINER EINEM PATIENTEN PERKUTAN EINZUFÜHRENDEN MEDINZINISCHEN NADELEINHEIT**
METHOD FOR PLANNING INTRACORPOREAL POSITIONING OF A MEDICAL NEEDLE UNIT TO BE INTRODUCED PERCUTANEOUSLY INTO A PATIENT
PROCÉDÉ POUR PRÉPARER LE POSITIONNEMENT INTRACORPOREL D'UNE UNITÉ FORMANT AIGUILLE MÉDICALE À INSÉRER PAR VOIE PERCUTANÉE CHEZ UN PATIENT

(30) Priorität: 25.05.2016 DE 102016209074
(43) Veröffentlichungstag der Anmeldung: 10.04.2019
(73) Patentinhaber: Medical Templates AG, 8132 Egg bei Zürich (CH)
(72) Erfinder: HOSTETTLER, Rafael, 80802 München (DE); WETZEL, Stephan, 8132 Egg bei Zürich (CH)
(74) Vertreter: Rösler, Uwe
(86) Internationale Anmeldenummer: PCT/EP2017/060887
(87) Internationale Veröffentlichungsnummer: WO 2017/202590

(56) Entgegenhaltungen:
- WO-A1-92/06645
- DE-A1- 19 805 112
- US-A- 5 943 719
- US-A1- 2003 036 766
- US-A1- 2008 146 963
- US-B1- 6 529 765
- , 28 October 2011 (2011-10-28), Retrieved from the Internet: URL:https://www.youtube.com/watch?v=fkvkeP ojVyU&feature=youtu.be [retrieved on 2020-01-10]

## Beschreibung

### Technisches Gebiet

Die Erfindung bezieht sich auf ein Verfahren zur Planung einer intrakorporalen Verortung einer einem Patienten perkutan einzuführenden medizinischen Nadeleinheit.

### Stand der Technik

Es gibt mehrere medizinische Situationen, in denen ein kontrolliertes Einführen und Positionieren einer Punktionsnadel an einer bestimmten Stelle im Körper eines Patienten erforderlich ist. Als Punktionsnadeln kommen insbesondere Biopsienadeln zur Entnahme von Gewebeproben zum Einsatz. Daneben kommen auch Injektionsnadeln zur kontrollierten Verabreichung einer Substanz in einer bestimmten Region im Körperinneren sowie Führungsnadeln für Operationsschrauben oder - bohrer in Frage. Gleichsam werden Punktionsnadeln im Rahmen medizinischer Therapien zur gezieltem Stimulation eingesetzt. Bei all diesen Anwendungen gilt es, einen definierten Teil der Punktionsnadel - primär deren Spitze - an einer gewünschten Stelle im Körperinneren zu positionieren. Darüber hinaus ist eine kontrollierte Einführung der Punktionsnadel erforderlich, d.h. auch die Einstichstelle an der Körperoberfläche, sowie der Stichkanal werden aufgrund verschiedener Kriterien gewählt.

Wenn eine möglichst präzise Einführung und/oder Positionierung der Punktionsnadel erforderlich ist, wird der Vorgang unter Zuhilfenahme von bildgebenden Verfahren durchgeführt. Zu diesem Zweck sind bereits mehrere Einführungs- und Positioniervorrichtungen vorgeschlagen worden. Als bildgebende Verfahren werden vornehmlich Röntgentomographie (CT), Fluoroskopie, 3D Röntgen, sowie die Magnetresonanztomografie (MRT)eingesetzt.

EP 0 640 842 A1 beschreibt eine MRT gestützte Biopsievorrichtung, die insbesondere für mammografische Untersuchungen vorgesehen ist. Die Vorrichtung umfasst eine mit einem Raster von Durchgangslöchern versehene Platte, welche eine x-y-Ebene definiert und durch welche eine Biopsienadel in z-Richtung vorgetrieben werden kann. Durch Wahl eines bestimmten Durchgangslochs für die Nadel ist eine grobe x-y-Positionierung möglich. Falls die x-y-Position genauer als der Lochabstand eingestellt werden soll, kann die ganze Platte mittels einer Stellvorrichtung kontinuierlich in der x-y-Ebene verschoben werden. Die Vorrichtung umfasst weiterhin mindestens ein stabförmiges Phantom, das in einer bekannten Ausrichtung gegenüber dem Gehäuse fixiert ist. Das Phantom besteht aus einem im MRT sichtbaren Material und dient als Positionierhilfe für die Biopsievorrichtung. Vorteilhafterweise ist das Phantom als orthogonales Kreuz ausgebildet, dessen Arme beispielsweise in x- bzw. y-Richtung verlaufen. In einer Ausführungsform ist zudem die Biopsienadel im Bereich der Spitze mit einem weiteren Phantom ausgestattet, mit dessen Hilfe die z-Position der Nadelspitze im MRT-Bild bestimmbar ist.

Auch die in WO 99/058069 beschriebene Punktionsvorrichtung für Schichtaufnahmeverfahren beruht auf der Verwendung von länglichen Hohlräumen, die mit einem entsprechend gewählten Kontrastmittel gefüllt sind. Dadurch, dass eine Mehrzahl solcher Hohlräume mit unterschiedlicher Richtung und Versatz vorhanden ist, lässt sich anhand der in einer Schichtaufnahme erkennbaren Schnitte der Hohlräume die Position und Neigung der Punktionsvorrichtung bestimmen.

EP 2409645 A1 sowie DE 10 2011 080 682 A1 betreffen weitere bildgebungsunterstützte Biopsievorrichtungen, welche eine steuerbare Einheit für die Positionierung und den Vortrieb einer Biopsienadel umfassen. Auch in diesen Fällen kommt eine Positioniereinheit zum Einsatz, die einen kontrollierten Nadelvortrieb in z-Richtung und eine Wahl der Nadelposition in einer dazu senkrechten x-y-Ebene erlaubt. Ein solcher Ansatz ist gerade bei mammografischen Biopsievorrichtungen zweckmässig, weil die zu untersuchende Körperregion zwischen zwei Platten angeordnet und somit pseudoflächig geformt ist. Dementsprechend ist der Zugang zu einer bestimmten Stelle vorteilhaft durch einen Einstich quer zur Plattenebene möglich. Es gibt jedoch zahlreiche andere Situationen, in denen ein direkter Einstich auf dem kürzesten Weg senkrecht zur Hautoberfläche wegen dazwischen liegender Strukturen nicht möglich oder zumindest suboptimal ist.

Einen Ansatz mit wählbarem Einstichwinkel ist in US 6249713 B1 beschrieben. Um die Spitze einer Biopsienadel von einer vorgegebenen Einstichstelle an eine gewünschte Stelle im Körperinnern zu bringen, kann die Richtung der Nadelachse eingestellt werden. Insbesondere kann diese Richtung in an sich bekannter Weise durch einen Polarwinkel und einen Azimutwinkel definiert werden. Allerdings ist die Prozedur wie auch die betreffende Vorrichtung vergleichsweise aufwändig.

Eine weitere Art von Biopsienadelführung ist in der Druckschrift US 2008/0146963 beschrieben. Diese umfasst zwei endständig miteinander verbundene bandförmige Elemente, die je mit einer Vielzahl von Durchgangslöchern für eine Biopsienadel versehen sind. Das erste bandförmige Element ist zum anliegenden Befestigen an einem Körperteil eines Patienten vorgesehen, wobei die einzelnen Durchgangslöcher entsprechende Einstichpositionen definieren. Das zweite bandförmige Element ist zwischen den endständigen Verbindungen länger als das erste Element und verläuft dementsprechend nach aufwärts gebogen. Bei gegebener Einstichposition lässt sich die Einstichrichtung durch Wahl eines Durchgangsloches im oberen Bandelement approximativ festlegen. Wegen der Biegsamkeit des oberen Bandelementes liegt jedoch keine eigentliche Führung vor, sondern vielmehr eine Ausrichtungshilfe.

Die Druckschrift US 2003/0036766 A1 offenbart ein Nadelführungsmittel zur intrakorporalen Platzierung einer Biopsienadelanordnung unter Sichtkontrolle mittels einer Magnetresonanzbildüberwachung, wobei das Nadelführungsmittel zwei jeweils mit Durchgangslöchern perforierte Platten umfasst, von denen eine erste Platte körpernah und eine zweite Platte parallel und beabstandet zur Ersten angeordnet sind. Die Durchgangsöffnungen innerhalb der ersten und zweiten Platte sind jeweils zum Durchführen der Biopsienadelanordnung entsprechend dimensioniert angepasst, wobei ein bestimmt gewählter Einstichkanal jeweils durch eine in der ersten und zweiten Platte liegende Durchgangsöffnung definiert wird.

In der Druckschrift US 2004/0143150 A1 ist eine vergleichbare Nadelführungsanordnung beschrieben, die aus einem gelochten Plattenpaar besteht und zur Trajektorienwahl für ein inkorporales Einführen einer medizinischen Nadeleinheit dient. Die bekannte Nadelführungsanordnung lässt sich lösbar ortsfest direkt am Patienten befestigen und stellt auf diese Weise ein extrakorporales, Patienten-integrales Nadelführungsmittel dar.

Das bekannte Nadelführungsmittel ermöglicht überdies ein wiederholtes Einführen der medizinischen Nadeleinheit an einer bestimmten Punktionsstelle längs eines bestimmt vorgegebenen Einstichkanals, solange das Nadelführungsmittel in einer festen Verbindung zum Patienten verbleibt.

Gleichwohl es möglich ist, den Punktionsvorgang, das heißt das Einführen der medizinischen Nadeleinheit in den Patienten, unter Sichtkontrolle, das heißt beispielsweise unter Verwendung eines Röntgenbild-gestützten Verfahrens oder Ähnlichem durchzuführen, kann es vorkommen, dass die Punktion korrigiert werden muss, so dass der Patient wenigstens ein zweites Mal zum Auffinden einer idealen Punktionstrajektorie zu belasten ist. Hinzu kommt, dass insbesondere beim Einsatz von röntgenbildgestützten Sichtkontrollen der Arzt einer Strahlenbelastung während der Punktion ausgesetzt ist.

Der US 6,366,796 B1 sind ein Verfahren und eine Vorrichtung zur Planung eines chirurgischen Eingriffes im Wege einer Brachytherapie zu entnehmen, beim dem ein Operateur mithilfe eines Nadelführungsmittels, das über eine Stativanordnung relativ zu einem Patienten platziert wird, sowie gestützt auf einer Vielzahl von auf einem Sichtgerät darstellbaren CT-Schnittbildaufnahmen von dem Patienten samt Nadelführungsmittel, eine ideale Punktionslage und -tiefe vor Einbringen der Nadel in den Patienten bestimmen kann. Dies erfolgt mithilfe einer in die CT-Schnittbilder projizierbaren virtuellen Projektionsnadel, die der Operateur durch das Nadelführungsmittel in Abhängigkeit der patientenspezifischen Gegebenheiten individuell positionieren kann. Eine weiterführende Unterstützung zur exakten Handhabung der Projektionsnadel erhält der Operateur nicht. So bedarf es einer großen Aufmerksamkeit des Operateurs, dass die Projektionsnadel korrekt durch das Nadelführungsmittel hindurchgeführt wird.

Vergleichbare Hinweise sind der US 6,529,765 B1 zu entnehmen, die überdies ein Verfahren zur ortsaufgelösten, maschinenunterstützten Handhabung eines chirurgischen Instrumentes relativ zu einem patientenseitig fest angebrachten Orientierungs- und Zentrierrahmens offenbart.

Die Druckschrift US 5,943,719 B1 offenbart ein Verfahren zur Planung einer intrakorporalen Verortung einer einem Patienten perkutan einzuführenden medizinischen Nadeleinheit unter Zugrundelegung eines Punktionsplanes.

### Darstellung der Erfindung

Der Erfindung liegt die Aufgabe zugrunde, einem zu behandelnden Arzt die Möglichkeit für eine Vorabbestimmung und Festlegung eines idealen Einstichkanals zum intrakorporalen Einbringen der medizinischen Nadeleinheit, zu schaffen, so dass sowohl die Patientenbelastung durch Vermeidung möglicher Punktionskorrekturen sowie auch die Arztbelastung bedingt durch eine Strahlenexposition vollständig vermieden werden können. Zudem soll sichergestellt werden, dass der Punktionsvorgang sicher und möglichst schnell am Patienten durchgeführt werden kann. Hierzu gilt es dem Arzt sämtliche Informationen für das intrakorporale Einbringen einer medizinischen Nadeleinheit in unmissverständlicher und kognitiv einfach und schnell erfassbarer Weise darzubieten, insbesondere in Fällen, in denen ein Nadelführungsmittel zum Einsatz kommt, das über ein große Vielzahl sehr eng zusammenliegender Nadeldurchstoßöffnungen verfügt.

Die Lösung der der Erfindung zugrundeliegenden Aufgabe ist im Anspruch 1 angegeben. Den Erfindungsgedanken in vorteilhafter Weise ausbildende Merkmale sind Gegenstand der Unteransprüche sowie der weiteren Beschreibung unter Bezugnahme auf die Ausführungsbeispiele zu entnehmen.

Lösungsgemäß wird ein Verfahren zur Planung einer intrakorporalen Verortung einer einem Patienten perkutan einzuführenden medizinischen Nadeleinheit unter Zugrundelegung eines Punktionsplanes angegeben, das sich durch die folgenden Verfahrensschritte auszeichnet:
In einem ersten Schritt wird ein Nadelführungsmittel raumfest am Patienten über einer Zielregion angeordnet und mit diesem fest verbunden. Vorzugsweise erfolgt die Verbindung des Nadelführungsmittels über eine adhäsiv wirkende Klebverbindung, so dass das Nadelführungsmittel schonend an die Hautoberfläche des Patienten adhäsiv anhaftend und von dieser ebenso für den Patienten schonend entfernbar anbringbar ist.

In einem zweiten Schritt wird eine Schar von Schnittbildern, jeweils enthaltend raumaufgelöste Bildinformationen vom Patienten sowie von dem mit dem Patienten raumfest verbundenen Nadelführungsmittel unter Verwendung eines bildgebenden diagnostischen Verfahrens generiert und entsprechend abgespeichert. Sämtliche mit Hilfe des bildgebenden diagnostischen Verfahrens gewonnenen Bildinformationen liegen als raumaufgelöste Bildinformationen innerhalb eines ersten Koordinatensystems vor, das von dem jeweils gewählten System zur Durchführung des bildgebenden diagnostischen Verfahrens festgelegt wird. Ebenso kann die Schar an Schnittbildern jeweils aus einem dreidimensionalen Bilddatensatz gewonnen werden, der von dem Patienten mit dem auf dem Patienten aufsitzenden Nadelführungsmittel gewonnen wurde. Grundsätzlich können aus dem Bilddatensatz auch nur zwei, drei, vier oder mehr geeignet gewählte Schnittbilder generiert werden, die für eine weitere Verwendung zur Verfügung stehen.

In einem nächsten Schritt wird wenigstens ein Schnittbild aus der Schar abgespeicherter Schnittbilder ausgewählt und visuell, vorzugsweise auf einem Monitor dargestellt. Alternativ ist es ebenso möglich das wenigstens eine Schnittbild numerisch zu generieren auf der Grundlage eines oder mehrerer abgespeicherter Schnittbilder. Die Auswahl des wenigstens einen Schnittbildes wird vorzugsweise durch einen zu behandelnden Arzt vorgenommen, dessen Aufgabe es ist, die medizinische Nadeleinheit zu Zwecken eines bestimmten diagnostischen oder therapeutischen Zielvorhabens innerhalb des Patienten in einem bestimmten Bereich zu platzieren.

In einem weiteren Verfahrensschritt wird dem ausgewählten und dargestellten Schnittbild eine virtuelle, positionsveränderliche Lineartrajektorie überlagert. Dieser Vorgang lässt sich vorzugsweise unter Verwendung eines geeigneten Bildverarbeitungsprogrammes durchführen, bei dem der Arzt durch manuelle Bedienung eines graphischen Eingabemittels, beispielsweise in Form einer Computermaus oder einer berührempfindlichen Benutzereingabeoberfläche, eine gegenüber dem auf einem Monitor dargestellten Schnittbild, sich kontrastreich abhebende, als Linie dargestellte Lineartrajektorie frei navigieren bzw. platzieren kann.

Auf der Grundlage eines bestimmt vorgegebenen Punktionsplanes wird die virtuelle Lineartrajektorie zum Erhalt einer Soll-Lineartrajektorie, in der die virtuelle Lineartrajektorie das Nadelführungsmittel traversiert, positioniert. Unter dem Begriff "Soll-Lineartrajektorie" ist jene, vorzugsweise durch den Arzt, frei wählbare Idealposition einer Lineartrajektorie relativ zu dem auf dem Monitor dargestellten Schnittbild zu verstehen, längs der die medizinische Nadeleinheit intrakorporal in den Patienten eingeführt werden soll. Die Soll-Lineartrajektorie repräsentiert somit den Einstichkanal für die medizinische Nadeleinheit.

In einem nachfolgenden Verfahrensschritt werden die Raumkoordinaten von zwei separaten Raumpunkten, den so genannten Traversierungspunkten, an denen die Soll-Lineartrajektorie das Nadelführungsmittel traversiert, in Form von Raumkoordinaten innerhalb des ersten Koordinatensystems ermittelt.

Hierzu sieht das lösbar fest am Patienten angebrachte Nadelführungsmittel wenigstens ein oberes, vom Patienten entfernt angeordnetes Plattenelement und ein dem Patienten zugewandt angeordnetes, unteres Plattenelement vor, die jeweils starr miteinander und voneinander beabstandet sind und jeweils eine Vielzahl von Durchgangslöchern zur Durchführung der medizinischen Nadeleinheit vorsehen.

Nachfolgend werden die das Nadelführungsmittel sowie zumindest die die Traversierungspunkte betreffenden Raumkoordinaten aus dem ersten in ein zweites Koordinatensystem transformiert, innerhalb dem in einem letzten Verfahrensschritt das Nadelführungsmittel mit den an dem Nadelführungsmittel visuell wahrnehmbar markierten Traversierungspunkten visuell dargestellt werden.

Im Rahmen der visuellen Darstellung des Nadelführungsmittels mit den an diesem als sichtbar wahrnehmbar kontrastreich hervortretenden Traversierungspunkten, vermag der Arzt die exakte Relativlage der Soll-Lineartrajektorie zum Nadelführungsmittel eindeutig zu erfassen, so dass er auf der Grundlage dieser ihm visuell vermittelten Informationen die medizinische Nadeleinheit zielsicher und schnell über das fest am Patienten verbundene Nadelführungsmittel an der vorgegebenen Position und mit der vorbestimmten Einstechkanalrichtung in den Patienten einführen kann.

Sämtliche zum Auffinden bzw. Bestimmen der Soll-Lineartrajektorie zu berücksichtigenden Maßnahmen, wie beispielsweise das Auffinden eines frei zugänglichen Einstichkanals durch die Hautoberfläche zu einem bestimmten intrakorporalen Bereich ohne Berührung bzw. Verletzung intrakorporaler Objekte, wie beispielsweise Organe, Gefäße, Knochen, etc., können vom Arzt getrennt vom Patienten in aller Ruhe und Sorgfalt an einem rechnergestützten, bildverarbeitenden Arbeitsplatz durchgeführt werden. Weder der Patient noch der betreffende Arzt sind Belastungen wie beispielsweise in Form von Justierpunktionen oder Strahlenbelastungen ausgesetzt.

Um das Auffinden der Soll-Lineartrajektorie für den Arzt zu erleichtern, sieht eine bevorzugte Weiterbildung des lösungsgemäßen Verfahrens vor, wenigstens zwei Schnittbilder gleichzeitig visuell wahrnehmbar darzustellen, die jeweils eine Patientenansicht und das Nadelführungsmittel aus unterschiedlichen Blickwinkeln wiedergeben. In beiden Schnittbilddarstellungen wird die vom Arzt manuell frei verschiebbare Lineartrajektorie jeweils in überlagernde Darstellung gebracht, anhand der es dem Arzt möglich ist, einen dreidimensionalen Eindruck über die Lage der virtuell dargestellten Lineartrajektorie relativ zu den räumlichen Gegebenheiten des Patienten kognitiv leicht erfassbar zu erhalten. Die zwei oder mehr jeweils unmittelbar nebeneinander dargestellten Schnittbilder können auf unterschiedlichen Monitoren oder in separat auf einem Monitor dargestellten Schnittbildflächen illustriert werden. Die Art, Größe und graphische Darstellungsform der jeweiligen Schnittbilder durch den Patienten sowie dem darauf fest angeordneten Nadelführungsmittel sowie die mit den Schnittbilder jeweils in Überlagerung gebrachte Lineartrajektorie ist unter Maßgabe einer vom Arzt sicher, eindeutig und schnell kognitiv erfassbaren Weise optimiert zu wählen.

Der Vorgang des Positionierens der virtuellen Lineartrajektorie erfolgt unter Zugrundelegung eines bestimmten Punktionsplanes, der zumindest die Anzahl der intrakorporal einzubringen medizinischen Nadeleinheiten sowie deren intrakorporale Position und Lage, insbesondere jeweils der distalen Nadelspitze, umfasst, und führt letztlich zum Erhalt der idealen Soll-Lineartrajektorie für jeweils eine medizinische Nadeleinheit, wobei die Soll-Lineartrajektorie jeweils durch ein Durchgangsloch des oberen und unteren Plattenelementes verläuft. Selbstverständlich können auch zwei oder mehr medizinische Nadeleinheiten zugleich über ein Nadelführungsmittel in den Patienten eingeführt werden. Zum Festlegen der den einzelnen medizinischen Nadeleinheiten zugeordneten Soll-Lineartrajektorien gilt zudem zu beachten, dass sie sich räumlich nicht durchdringen.

Die graphische Darstellung des Nadelführungsmittels auf einem Schnittbild erfolgt im einfachsten Fall durch die im Rahmen des bildgebenden diagnostischen Verfahrens gewonnenen räumlich aufgelösten Bildinformationen. In einer bevorzugten Verfahrensvariante wird das Nadelführungsmittel unter Zugrundlegung eines an sich bekannten CAD-Datensatzes, der die exakte Raumform des Nadelführungsmittels repräsentiert, numerisch generiert und größen- und raumwinkelgetreu zur Schnittbilddarstellung in Überlagerung gebracht. Hierzu wird ein raumfest am Nadelführungsmittel angebrachter Marker, der sich möglichst kontrastreich in den generierten und abgespeicherten Schnittbildern darstellt, unter Verwendung eines numerischen Mustererkennungsprogramms lageaufgelöst innerhalb des ersten Koordinatensystems erfasst. Unter Zugrundelegung des ortsaufgelösten Markers werden räumlich aufgelöste Bildinformationen des Nadelführungsmittels synthetisch generiert und dem Schnittbild graphisch überlagert. Auf diese Weise wird eine hochgenaue raumaufgelöste, größen- und raumwinkelgetreue Darstellung des Nadelführungsmittels auf den jeweils visuell dargestellten Schnittbildern generiert.

Auf der Grundlage der das Nadelführungsmittel visuell wiedergebenden Bildinformationen sowie den die Soll-Lineartrajektorie bestimmenden Raumkoordinaten werden mit Hilfe eines geeigneten Bildauswerteprogrammes die Raumkoordinaten der Traversierungspunkte ermittelt, an denen die Soll-Lineartrajektorie das Nadelführungsmittel traversiert. Im Falle der Verwendung des vorstehend bereits erläuterten Nadelführungsmittels, umfassend wenigstens das obere und untere Plattenelement, kennzeichnen die Traversierungspunkte jene Durchgangslöcher innerhalb des oberen und unteren Plattenelementes, durch die die Soll-Lineartrajektorie hindurchragt.

Ziel ist es dem Arzt jene Informationen zu vermitteln, die er benötigt eine reale medizinische Nadeleinheit derart in das real auf dem Patienten raumfest angebrachte Nadelführungsmittel einzuführen, so dass die medizinische Nadeleinheit in exakt der gleichen räumlichen Orientierung und Lage durch das reale Nadelführungsmittel zu Zwecken der Patientenpunktion hindurchgeführt wird, in der die virtuell dargestellte Soll-Lineartrajektorie das auf dem Bildschirm wiedergegebene virtuelle Nadelführungsmittel traversiert.

Um diese Information dem Arzt in einer leicht kognitiv zugänglichen und fehlerfreien Weise zu vermitteln, wird das auf dem jeweiligen Schnittbild dargestellte Nadelführungsmittel samt der dieses traversierenden Soll-Lineartrajektorie in einer separaten Darstellung wiedergegeben. Hierzu ist es erforderlich, die räumlichen Koordinaten sämtlicher Bildinformationen des Nadelführungsmittels sowie zumindest die Raumkoordinaten der zwei Traversierungspunkte aus dem ersten in ein zweites Koordinatensystem zu transformieren. Alternativ besteht die Möglichkeit anstelle der Transformation der Raumkoordinaten sämtlicher das Nadelführungsmittel darstellenden Bildinformationen lediglich die Raumkoordinaten des detektierten wenigstens einen Markers des Nadelführungsmittels in das zweite Koordinatensystem zu transformieren. Für eine gesamtheitliche virtuelle Darstellung des Nadelführungsmittels innerhalb des zweiten Koordinatensystems werden auf die bekannten, die Raumform des Nadelführungsmittels beschreibenden Daten, vorzugsweise in Form von CAD-Daten, zurückgegriffen. Hierdurch kann der für die Transformation erforderliche Rechenaufwand reduziert werden, wodurch Rechenzeiten verkürzt und gegebenenfalls Rechenkapazitäten eingespart werden können. Sinn und Zweck der Koordinatentransformation in das zweite Koordinatensystem ist eine separate Darstellung des Nadelführungsmittels mit den zwei Traversierungspunkten. Durch eine geeignete virtuelle Darstellung kann der Arzt die genaue Örtlichkeit der Traversierungspunkte studieren. Beispielsweise ist es möglich, das Nadelführungsmittel samt Traversierungspunkte in einer dreidimensionalen Darstellung durch eine entsprechend nutzerspezifische Eingabe räumlich zu drehen, um die Traversierungspunkte leicht zu erkennen.

Vorzugsweise wird die visuelle Darstellung des Nadelführungsmittel mit den an dem Nadelführungsmittel visuell wahrnehmbar markierten Traversierungspunkten innerhalb des zweiten Koordinatensystems derart vorgenommen, so dass im Falle eines Nadelführungsmittels der vorstehend bezeichneten Art das obere und untere Plattenelement separat, jeweils in Draufsicht wiedergegeben werden, wobei an den Plattenelementen jeweils die Durchgangslöcher eindeutig lokalisierende Informationen sichtbar dargestellt werden. In einer bevorzugten Ausführungsform sind die Durchgangslöcher in Reihen und Spalten unterteilt, so dass eine eindeutige Durchgangslochspezifikation durch ein konkretes Wertepaar vorgenommen werden kann. Vorzugsweise eignen sich hierzu alphanumerische Zeichen zur Benennung der längs einer Spalte sowie einer Zeile verteilten Durchgangslöcher. Selbstverständlich sind auch alternative Kennzeichnungen zur Spezifikation der einzelnen Traversierungspunkte denkbar.

In Kenntnis dieser die Durchgangslöcher spezifizierenden Informationen, kann der Arzt die reale medizinische Nadeleinheit durch das reale Nadelführungsmittel hindurchführen.

Das lösungsgemäße Verfahren ermöglicht anhand der Erkennung des am Nadelführungsmittel angebrachten wenigstens einen Markers eine Verifikation des verwendeten Nadelführungsmittels, indem der erfasste Marker mit entsprechend in einer Datenbank bzw. Bibliothek abgelegten Referenzmarker-Daten verglichen wird. Bei Übereinstimmung ist das tatsächlich eingesetzte Nadelführungsmittel erkannt und kann einem Datensatz zur Beschreibung der vollständigen Raumform des betreffenden Nadelführungsmittels zugeordnet werden. Ein derartiger Datenabgleich ermöglicht im Rahmen der Verifikation das Erkennen von möglichen Plagiaten. Darüber hinaus ermöglicht der Datenabgleich eine automatische Zuordnung des jeweils eingesetzten Nadelführungsmittels zu einer bestimmten Kategorie von Nadelführungsmitteln, die sich bspw. jeweils in Form und Größe voneinander unterscheiden.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:

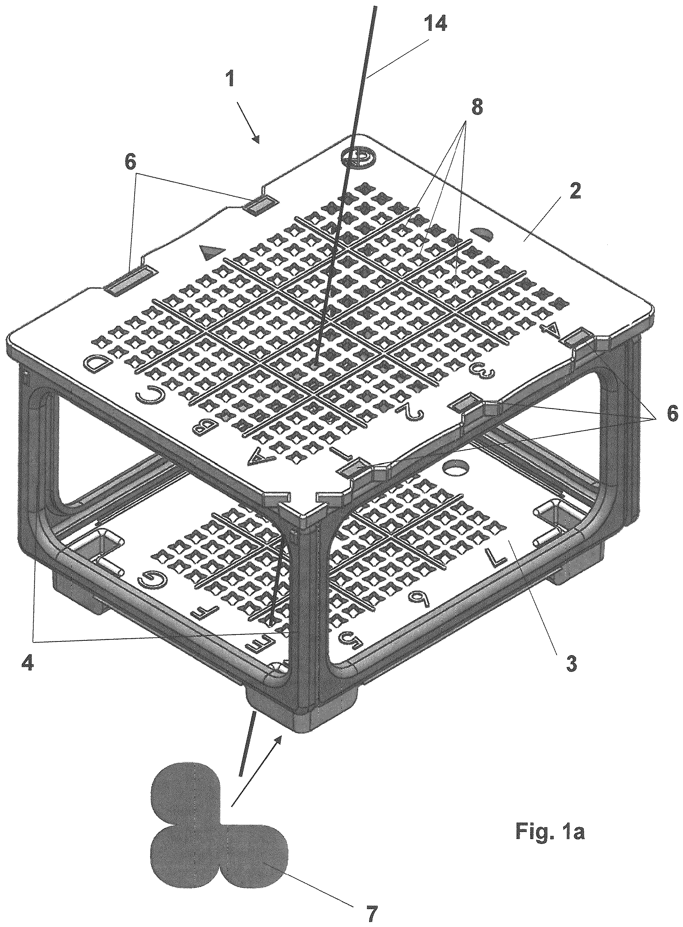

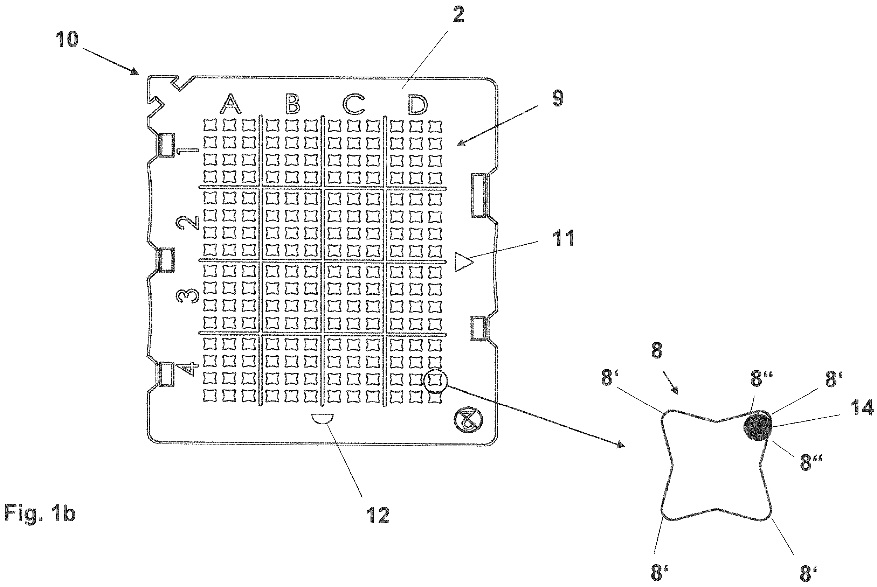

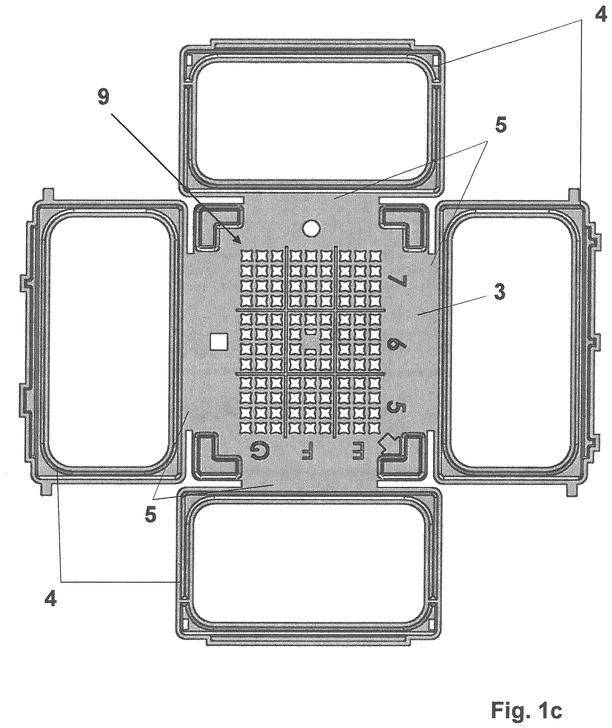

- Fig. 1 a: Gesamtdarstellung eines bevorzugten Nadelführungsmittels,
- Fig. 1 b: Draufsicht auf das obere Plattenelement des Nadelführungsmittels,
- Fig. 1 c: Draufsicht auf das untere Plattenelement mit Tragstrukturen des Nadelführungsmittels
- Fig. 2 a, b: NMR-Schnittbilder durch einen Patienten mit Nadelführungsmittel und überlagerter Lineartrajektorie sowie
- Fig. 3 a, b: Draufsichtdarstellungen des oberen und unteren Plattenelementes mit Traversierungspunkten

### Wege zur Ausführung der Erfindung, gewerbliche Verwendbarkeit

In Figur 1a ist in einer perspektivischen Gesamtdarstellung ein bevorzugtes Nadelführungsmittel 1 dargestellt, das im Wesentlichen aus einem oberen Plattenelement 2 sowie einem unteren Plattenelement 3 besteht, die beide parallel zueinander gelagert und über eine Tragstruktur 4 lateral voneinander beabstandet angeordnet sind. In Figur 1b sind das obere Plattenelement 2 in Draufsicht sowie in Figur 1c das untere Plattenelement 3 ebenfalls in Draufsicht mit den daran angelenkten Tragstrukturen 4 dargestellt. Zur weiteren Beschreibung betreffend das Nadelführungsmittel 1 sei auf die Figuren 1a bis c wird gemeinschaftlich Bezug genommen.

Das in Figur 1a dargestellte Nadelführungsmittel 1 ist aus einem biokompatiblen Material, vorzugsweise einem sterilisierbaren Kunststoffmaterial gefertigt, das den Einsatz in Sterilräumen mit unmittelbaren Patientenkontakt erlaubt. Vorzugsweise ist das Nadelführungsmittel 1 im Wege eines Urformverfahrens oder generativen Herstellungsverfahrens gefertigt. Im Wesentlichen besteht das Nadelführungsmittel 1 aus dem oberen Plattenelement 2 sowie dem unteren Plattenelement 3. Beide Plattenelement 2, 3 sind über eine einstückig mit dem unteren Plattenelement 3 verbundene Tragstruktur 4 beabstandet. Die Tragstruktur 4 ist vorzugsweise über Scharniergelenke 5 mit dem unteren Plattenelement e gelenkig um jeweils eine Achse schwenkbar verbunden. Beide Plattenelemente 2, 3 sind über geeignet ausgebildete Rastverbindungen 6 lösbar fest verbunden und stellen im zusammengefügten Zustand eine quader- oder würfelförmige Grundform dar. Die Rastverbindungen 6 sind derart ausgebildet und angeordnet, dass ein Zusammenfügen beider Plattenelemente 2, 3 nur in einer einzigen eindeutigen Zuordnung möglich ist.

Zu Zwecken einer raumfesten Fixierung des Nadelführungsmittels 1 an der Hautoberfläche eines Patienten (nicht dargestellt) sind am unteren Plattenelement 3 geeignet ausgebildete Adhäsionsmittel 7 vorgesehen, die in Form von Klebepads eine adhäsive temporäre Verbindung mit der Haut des Patienten herstellen. Die Form der Klebepads 7 ist vorzugsweise derart gewählt, dass sie möglichst faltenfrei an unterschiedlichen Körperkonturen anlegbar sind. Als Material eignet sich vorzugsweise PVC-Schaum, der einseitig mit einem biokompatiblen Klebstoff für die Anwendung auf der Haut beschichtet ist.

Die Hauptfunktion des Nadelführungsmittels 1 besteht darin, Position und Lage einer medizinischen Nadeleinheit 14, die intrakorporal in einen Patienten durch die Haut eingeführt ist, räumlich zu bestimmen. Hierzu dient das auf einem Patienten raumfest anhaftende Nadelführungsmittel 1, durch das eine medizinische Nadeleinheit 14 zu Zwecken der Patientenpunktion hindurchgeführt wird. Hierbei durchstößt die Nadeleinheit 14 sowohl das obere als auch untere Plattenelement 2, 3, die jeweils über eine Vielzahl geordnet angeordnete Durchgangsöffnungen 8 verfügen. Im Ausführungsbeispiel verfügt das obere Plattenelement 2 über 144 Durchgangsöffnungen 8 und das untere Plattenelement 3 über 82 Durchgangsöffnungen 8. Die Durchgangsöffnungen pro Plattenelement liegen sehr dicht nebeneinander. Typischerweise beträgt der gegenseitige Abstand der Öffnungsmittelpunkte zweier benachbarter Durchgangsöffnungen ca. 2 mm bis 7 mm. Somit kann die räumliche Lage der Nadeleinheit 14 durch zwei Durchgangsöffnungen charakterisiert werden, nämlich durch eine Durchgangsöffnung innerhalb des oberen Plattenelementes 2 sowie durch eine Durchgangsöffnung innerhalb des unteren Plattenelementes 3, durch die jeweils die medizinische Nadeleinheit hindurchragt.

Das im Weiteren beschriebene lösungsgemäße Verfahren dient dem jeweiligen Anwender, vorzugsweise einem Arzt zur exakten kognitiven Erfassung der beiden Durchgangsöffnungen, durch die die medizinische Nadeleinheit hindurchtritt. Hierzu dienen insbesondere geeignet ausgebildete Markierungen und Beschriftungen, die sowohl am oberen als auch unteren Plattenelement 2, 3 angebracht sind.

Jede der beiden Plattenelemente 2, 3 weist eine Vielzahl geeignet ausgebildeter Durchgangsöffnungen 8 auf, die jeweils ein Durchführen der medizinischen Nadeleinheit 14 durch das Nadelführungsmittel 1 ermöglichen und darüber hinaus eine sichere Führung und Positionierung der Nadeleinheit 14 innerhalb des Nadelführungsmittel sicherstellen. Die Form der Durchgangsöffnungen 8 ist in dem gezeigten Ausführungsbeispiel sternförmig ausgebildet und bietet für die medizinische Nadeleinheit 14 vier Stützstellenbereiche 8', siehe Detaildarstellung in Figur 1b, deren Geometrie so gewählt ist, dass eine medizinische Nadeleinheit 14 stets von zwei sich gegenüberliegenden Flanken 8" geführt und innerhalb des jeweiligen Stützstellenbereiches 8' zentriert wird. Auf diese Weise wird ein versehentliches Verrutschen der medizinischen Nadeleinheit 14 innerhalb einer Durchgangsöffnung 8 weitestgehend unterbunden. Zudem wird das Risiko vermindert, dass die medizinische Nadeleinheit 14 an einem anderen Stützstellenbereich 8' innerhalb der Durchgangsöffnung 8 zur Anlage kommt. Selbstverständlich sind alternative Geometrien für die Ausbildung der Durchgangsöffnungen denkbar. Um die Anzahl der möglichen Stützstellenbereiche 8' innerhalb einer Durchgangsöffnung 8 zu steigern, können mehr als vier Platzierungsmöglichkeiten für die medizinische Nadeleinheit vorgesehen werden, indem bspw. die Anzahl der sternförmigen Ecken vergrößert wird.

Die einzelnen Durchgangsöffnungen 8 sind sowohl im oberen Plattenelement 2 als auch im unteren Plattenelement 3 jeweils zu Feldern 9 zusammengefasst, wodurch eine bessere Orientierung und Wiederauffindung einer einzelnen Durchgangsöffnung 8 möglich wird. Jedes Feld 9 besteht aus zwölf Durchgangsöffnungen 8 und beinhaltet somit insgesamt 48 unterschiedliche Stützstellenbereiche 8'. Angeordnet sind die Durchgangsöffnungen 8 innerhalb eines Feldes 9 jeweils in drei Spalten und vier Reihen, wodurch das Feld 9 eine räumliche Orientierung erhält. Der Arzt kann auf diese Weise einfach innerhalb eines Feldes 9 aufgrund der räumlichen Orientierung eine Durchgangsöffnung 8 leichter identifizieren. Auch die Identifizierung eines bestimmten Stützstellenbereiches 8' wird durch die Feldanordnung der Durchgangsöffnungen leichter möglich.

Das obere Plattenelement weist sechzehn Felder 9 auf, die jeweils geometrisch und optisch wahrnehmbar einen seitlichen Abstand zueinander aufweisen. Die sechzehn Felder 9 sind somit in vier Reihen und vier Spalten angeordnet. Insgesamt verfügt das obere Plattenelement 3 über 768 Stützstellenbereiche 8'.

Demgegenüber weist das untere Plattenelement 3 lediglich neun Felder 9 auf, die jeweils in drei Reihen und drei Spalten angeordnet sind. Somit weist das untere Plattenelement 3 lediglich 428 Stützstellenbereich 8' auf.

Zu Zwecken einer leichten und eindeutigen Feldzuordnung sind die in Spalten und Reihen angeordneten Felder 9 mit alphanumerischen Zeichen markiert. So ist jede Feldspalte mit einem Buchstaben (A bis D bezüglich des oberen Plattenelementes 2 sowie E bis G bezüglich des unteren Plattenelementes 3) sowie jede Feldzeile mit einer Zahl bezeichnet (1 bis 4 bezüglich des oberen Plattenelementes 2 sowie 5 bis 7 bezüglich des unteren Plattenelementes 3). Die alphanumerischen Markierungen sind jeweils auf die Oberfläche der beiden Plattenelemente 2, 3 aufgeprägt, so dass sie optisch deutlich hervortreten. Ein bestimmtes Feld 9 wird demzufolge durch eine Kombination aus den Buchstaben der jeweiligen Spalte und Zahl der jeweiligen Zeile eindeutig identifiziert.

Ferner verfügt zumindest das obere Plattenelement 2 über eine Markierung 10, die in Form eines Pfeils ausgebildet ist. Die Markierung 10 erlaubt eine eindeutige Identifizierung der räumlichen Orientierung des oberen Plattenelementes 2. Die Markierung 10 ist ein einmalig vorkommendes Merkmal, wodurch die Bestimmung der Orientierung eindeutig ist. Die Markierung 10 ist in einer Ecke des oberen Plattenelementes 2 angeordnet und hebt sich dadurch optisch deutlich hervor.

Durch eine asymmetrische Ausbildung und Anordnung der Rastverbindungen 6 zwischen dem oberen Plattenelement 2 und dem unteren Plattenelemente 3 kann eine verdrehte und unkorrekte Anordnung beider Plattenelemente 2, 3 relativ zueinander sicher ausgeschlossen werden. Insofern dient die räumliche Erfassung der Markierung 10 auch der Bestimmung der räumlichen Anordnung und Ausrichtung des gesamten Nadelführungsmittels 1.

Ferner verfügt das obere Plattenelement 2 über weitere Markierungen 11, 12, die aufgrund ihrer Dreiecks- bzw. Halbkreisform mittels numerischer Mustererkennung im Rahmen einer Bilddatenauswertung lokalisiert und detektiert werden können. Die Markierungen 11, 12 dienen sowohl einer automatischen Lage- und Orientierungserkennung des Nadelführungsmittels 1 im Rahmen eines Bildauswerteverfahrens, sie dienen jedoch auch zusätzlich als optische Referenzpunkte zur leichteren, sicheren und schnelleren Erfassung einer bestimmten Durchgangsöffnung 9 am oberen Plattenelement 2 für einen Arzt.

Im Weiteren wird die Anwendung des vorstehend erläuterten Nadelführungsmittels 1 vorausgesetzt, das in der vorstehenden Ausbildung an der Hautoberfläche eines Patienten fest angebracht ist. Der Patient wird einem bildgebenden Diagnostizierverfahren unterzogen, beispielsweise ein NMR- oder CT-Verfahren, das Schnittbilder durch den Patienten sowie auch durch das Nadelführungsmittel 1 liefert.

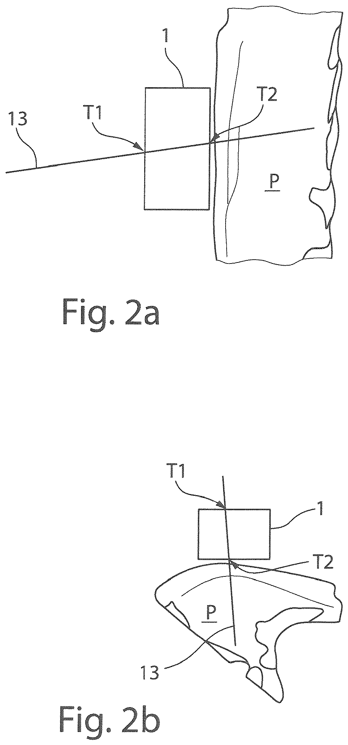

In den Figuren 2 a und b sind typische CT-Bildaufnahmen in Form von Schnittbilddarstellungen gezeigt, die sowohl ein Schnittbild durch den Patienten P als auch ein Schnittbild durch das auf dem Patienten P aufsitzende Nadelführungsmittel 1 zeigten und den Patienten und das Nadelführungsmittel unter unterschiedlichem Blickwinkel wiedergeben. Anhand der beiden unterschiedlichen Blickwinkel auf ein und denselben intrakorporalen Bereich innerhalb des Patienten P vermag sich der Arzt eine räumliche Vorstellung der Betrachtungssituation zu machen. Die jeweils nebeneinander auf einem Monitor dargestellten Schnittbilder ermöglichen dem Arzt überdies eine Überlagerung der Schnittbilder mit einer frei positionierbaren Lineartrajektorie 13, deren räumliche Ausrichtung relativ zum Patienten P durch den Arzt mit Hilfe eines geeigneten Eingabemittels, beispielsweise mittels Computermaus, beliebig variierbar ist. Die sich kontrastreich über den dargestellten Schnittbildern abzeichnende Lineartrajektorie 13 kann durch den Arzt so platziert werden, so dass eine nur minimale Belastung für den Patienten P während einer nachfolgenden Punktion auftritt. Beim Auffinden eines idealen Einstichkanals, der einer so genannten Soll-Lineartrajektorie 13 entspricht, durchragt die Soll-Trajektorie 13 das ebenfalls in den Schnittbildern dargestellte Nadelführungsmittel 8, dies ist in den Bildern gem. Fig. 2a, b der Fall. Anhand der dargestellten Schnittbilder kann der Arzt jedoch nicht ersehen, durch welche Durchgangsöffnungen 8 die Soll-Lineartrajektorie 13 jeweils innerhalb des oberen und unteren Plattenelementes 2, 3 hindurchragt.

Zu Zwecken der exakten Identifikation der Traversierungspunkte T1, T2 der Soll-Lineartrajektorie 13 durch das Nadelführungsmittel 1 erfolgt zunächst eine numerische Ermittlung der Raumkoordinaten der zwei Traversierungspunkte T1, T2 innerhalb des von Seiten der bildgebenden Aufnahmetechnik festgelegten ersten Koordinatensystems, innerhalb dem sämtliche Bildinformationen räumlich aufgelöst vorliegen. Um dem Arzt jedoch die Information zu vermitteln durch welche Durchgangsöffnungen 8 die Soll-Lineartrajektorie 13 jeweils längs des oberen und unteren Plattenelementes 2, 3 hindurchragt und insbesondere an welchen Stützstellenbereichen 8' die medizinische Nadeleinheit 14 zur Anlage kommen soll, gilt es die Traversierungspunkte T1, T2 sowie die Darstellung des Nadelführungsmittels 1 in ein zweites Koordinatensystem zu transformieren.

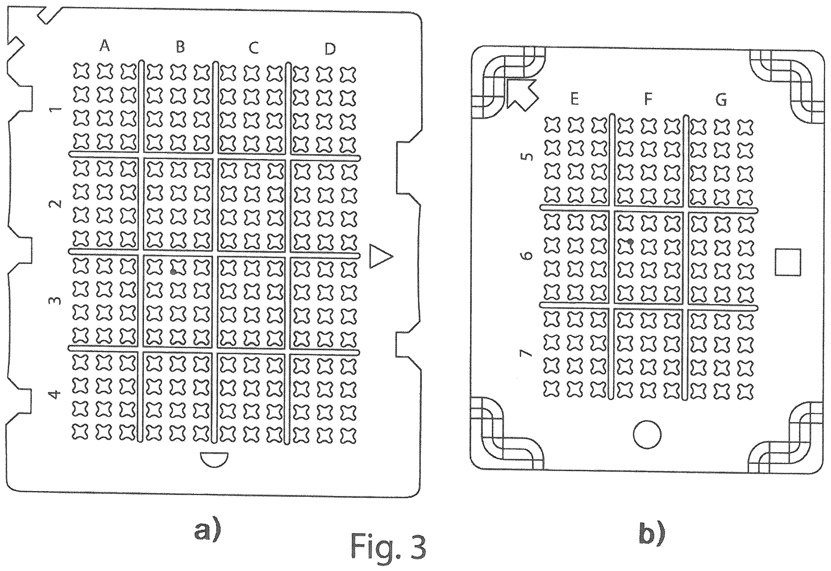

Zur visuellen Darstellung wird das Nadelführungsmittel vorzugsweise in zwei nebeneinander liegenden Schnittbildern dargestellt. Figur 3a zeigt die Draufsicht auf das obere Plattenelement 2, Figur 3b zeigt die Draufsicht auf das untere Plattenelement 3. In beiden Plattenelementen ist jeweils der Durchstoßpunkt bzw. der Traversierungspunkt T1, T2 kontrastreich hervorgehoben. Im dargestellten Fall traversiert die Soll-Lineartrajektorie das obere Plattenelement 2 durch eine Durchgangsöffnung 8, die im Feld B3 liegt und dort in der Durchgangsöffnung oberste Zeile mittlerer Spalte. Der Stützstellenbereich 8' befindet sich unten links innerhalb der Durchgangsöffnung. Im Falle des unteren Plattenelementes 3 befindet sich der Traversierungspunkt im Feld F6 und hier in der Durchgangsöffnung zweite Zeile, rechte Spalte. Der Stützstellenbereich in dieser Durchgangsöffnung ist oben rechts.

Der vorstehenden Beschreibung für eine schnelle und sichere, d.h. fehlerfreie Erfassung der Traversierungspunkte für den Arzt liegt ein dreistufiges Beschreibungskonzept zugrunde: In einem Schritt wird ein Traversierungspunkt pro Plattenelement durch die Angabe charakterisiert, in welchem Feld 9 der Traversierungspunkt liegt. Diese Charakterisierung erfolgt durch eine Buchstaben/Zahlen-Kombination, B3 für das obere Plattenelement und F6 für das untere Plattenelement. Eine Verwechslung zwischen beiden Plattenelementen ist zudem ausgeschlossen, da sich die Buchstaben und Zahlen pro Plattenelement nicht wiederholen.

In einem zweiten Schritt wird der jeweilige Traversierungspunkt innerhalb des betreffenden Feldes 9 charakterisiert. Im oberen Plattenelement befindet sich der Traversierungspunkt innerhalb des Feldes 9 in der ersten Zeile, zweite Spalte, d.h. kurz (Z1, S2); im unteren Plattenelement befindet sich der Traversierungspunkt innerhalb des betreffenden Feldes 9 in der zweiten Zeile, erste Spalte, d.h. kurz (Z2, S1).

Im dritten Schritt wird der Traversierungspunkt durch den Stützstellenbereich 8' innerhalb der jeweiligen Durchgangsöffnung 8 charakterisiert. Im oberen Plattenelement befindet sich der Traversierungspunkt innerhalb der Durchgangsöffnung 8 in der unteren linken Ecke, d.h. kurz "unten links"; im unteren Plattenelement befindet sich der Traversierungspunkt innerhalb der betreffenden Durchgangsöffnung 8 in der oberen rechen Ecke, d.h. kurz "oben rechts".

Somit ergibt sich für die exakte Lagebeschreibung beider Traversierungspunkte folgende Koordinatenbeschreibungen: (B3/Z1,S2/unten links; F6/Z2,S1/oben rechts). In einer bevorzugten Form werden diese Koordinatenangaben auf einem Monitor visuell angezeigt und/oder akustisch wahrnehmbar umgesetzt.

Die gesamte Bilddatenprozessierung erfolgt derart, so dass die Darstellungen gemäß der Figuren 2a, b und Figuren 3a, b dem Arzt simultan zur Ansicht gebracht werden, so dass der Arzt beim Auffinden der Soll-Lineartrajektorie stets beurteilen kann, ob die Soll-Lineartrajektorie auch tatsächlich durch jeweils eine Durchgangsöffnung innerhalb des oberen und unteren Plattenelementes hindurchragt. Sollte die kontrastreich dargestellte Lineartrajektorie außerhalb der jeweiligen Durchgangsöffnungen liegen, so muss der Arzt entsprechend nachjustieren.

### Bezugszeichenliste

- 1: Nadelführungsmittel
- 2: oberes Plattenelement
- 3: unteres Plattenelement
- 4: Tragstruktur
- 5: Filmscharniergelenk
- 6: Rastvorrichtung
- 7: Adhäsionsmittel
- 8: Durchgangsöffnung
- 8': Stützstellenbereich
- 8": Flanken
- 9: Feld
- 10: Markierung
- 11: Markierung
- 12: Markierung
- 13: Lineartrajektorie, Soll-Lineartrajektorie
- 14: Medizinische Nadeleinheit
- P: Patient
- T1, T2: Traversierungspunkte

## Patentansprüche

1. Verfahren zur Planung einer intrakorporalen Verortung einer einem Patienten perkutan einzuführenden Punktionsnadel (14) unter Zugrundelegung eines Punktionsplanes umfassend die folgenden Verfahrensschritte:
a) Anordnen eines Nadelführungsmittels (1) raumfest am Patienten,
b) Generieren und Abspeichern von einer Schar von Schnittbildern, jeweils enthaltend raumaufgelöste Bildinformationen vom Patienten sowie von dem mit dem Patienten raumfest verbundenen Nadelführungsmittel (1) in einem ersten Koordinatensystem, unter Verwendung eines bildgebenden diagnostischen Verfahrens,
c) Auswählen und visuelles Darstellen wenigstens eines Schnittbildes oder eines numerisch generierten Schnittbildes aus der Schar der abgespeicherten Schnittbilder,
d) Überlagern einer virtuellen positionsveränderlichen Lineartrajektorie (13) unter Zugrundelegung des ausgewählten Schnittbildes,
e) Positionieren der virtuellen Lineartrajektorie unter Zugrundelegung des Punktionsplanes zum Erhalt einer Soll-Lineartrajektorie, in der die virtuelle Lineartrajektorie (13) das Nadelführungsmittel (1) traversiert,
f) numerisches Ermitteln von Raumkoordinaten von zwei separaten Raumpunkten, den sogenannten Traversierungspunkten (T1, T2), innerhalb des ersten Koordinatensystems, an denen die Soll-Lineartrajektorie (13) das Nadelführungsmittel (1) traversiert,
g) Transformieren von das Nadelführungsmittel (1) sowie die Traversierungspunkte (T1, T2) betreffenden Raumkoordinaten in ein zweites Koordinatensystem,
**dadurch gekennzeichnet, dass** das Nadelführungsmittel (1) wenigstens ein oberes, vom Patienten entfernt angeordnetes Plattenelement (2) und ein dem Patienten zugewandt angeordnetes, unteres Plattenelement (3) umfasst, die starr voneinander beabstandet sind und jeweils eine Vielzahl von Durchgangslöchern (8) zur Durchführung einer Punktionsnadel (14) vorsehen,
dass das Positionieren der virtuellen Lineartrajektorie (13) unter Zugrundelegung des Punktionsplanes zum Erhalt der Soll-Lineartrajektorie (13) derart durchgeführt wird, dass die Soll-Lineartrajektorie (13) jeweils durch ein Durchgangsloch (8) des oberen und unteren Plattenelementes (2, 3) verläuft,
und
dass das Nadelführungsmittel (1) innerhalb des zweiten Koordinatensystems derart visuell dargestellt wird, dass die Traversierungspunkte (T1, T2) an dem Nadelführungsmittel (1) visuell wahrnehmbar markiert dargestellt werden.

2. Verfahren nach Anspruch 1,
**dadurch gekennzeichnet, dass** zumindest die Verfahrensschritte c) bis e) derart durchgeführt werden, so dass wenigstens zwei Schnittbilder visuell gleichzeitig wahrnehmbar dargestellt werden, die die virtuelle Lineartrajektorie (13) sowie den Patienten zusammen mit dem Nadelführungsmittel (1) aus jeweils unterschiedlichen Blickwinkeln zeigen.

3. Verfahren nach Anspruch 1 oder 2,
**dadurch gekennzeichnet, dass** das visuelle Darstellen des Nadelführungsmittels (1) innerhalb des zweiten Koordinatensystems derart erfolgt, so dass jeder Traversierungspunkt (T1, T2) in einem separaten Schnittbild durch das Nadelführungsmittel (1) dargestellt wird.

4. Verfahren nach einem der Ansprüche 1 bis 3,
**dadurch gekennzeichnet, dass** das Generieren der raumaufgelösten Bildinformationen von dem mit dem Patienten raumfest verbundenen Nadelführungsmittel (1) durch numerische Mustererkennung wenigstens eines am Nadelführungsmittel (1) angebrachten Markers (10, 11, 12) erfolgt, dessen räumliche Position und Lage innerhalb des ersten Koordinatensystems bestimmt wird.

5. Verfahren nach Anspruch 4,
**dadurch gekennzeichnet, dass** unter Rückgriff auf die Raumform des Nadelführungsmittels (1) beschreibenden Daten und unter Zugrundelegung der Position und Lage des wenigstens einen Markers (10, 11, 12) innerhalb des ersten Koordinatensystems die raumaufgelösten Bildinformationen des Nadelführungsmittels (1) synthetisch generiert werden.

6. Verfahren nach Anspruch 4 oder 5,
**dadurch gekennzeichnet, dass** zu Zwecken der Transformation der wenigstens eine am Nadelführungsmittel (1) angebrachte Marker (10, 11, 12) numerisch detektiert und dessen Raumkoordinaten im ersten Koordinatensystem lokalisiert werden, und dass auf der Grundlage der lokalisierten Raumkoordinaten des wenigstens einen Markers (10, 11, 12) die Bildinformationen des Nadelführungsmittels (1) betreffend sowie die ermittelten Raumkoordinaten der zwei Traversierungspunkte (T1, T2) in das zweite Koordinatensystem transformiert werden.

7. Verfahren nach einem der Ansprüche 1 bis 6,
**dadurch gekennzeichnet, dass** das wenigstens eine, visuell dargestellte, numerisch generierte Schnittbild unter Zugrundlegung wenigstens einer Auswahl von mit Hilfe des bildgebenden diagnostischen Verfahrens aufgenommenen Schnittbildern berechnet wird.

8. Verfahren nach einem der Ansprüche 1 bis 7,
**dadurch gekennzeichnet, dass** nach Erhalt der Soll-Lineartrajektorie (13) die Raumkoordinaten der zwei Traversierungspunkte (T1, T2) durch das Nadelführungsmittel (1) unter Zugrundelegung der bekannten Bildinformation des Nadelführungsmittels (1) innerhalb des ersten Koordinatensystem ermittelt werden.

9. Verfahren nach einem der Ansprüche 1 bis 8,
**dadurch gekennzeichnet, dass** das visuelle Darstellen des Nadelführungsmittels (1) mit den an dem Nadelführungsmittel (1) visuell wahrnehmbar markierten Traversierungspunkten (T1, T2) innerhalb des zweiten Koordinatensystems derart erfolgt, so dass das obere und untere Plattenelement (2, 3) separat, jeweils in Draufsicht wiedergegeben werden, wobei an den Plattenelementen (2, 3) jeweils die Durchgangslöcher (8) eindeutig lokalisierende Informationen sichtbar dargestellt werden.

10. Verfahren nach Anspruch 9,
**dadurch gekennzeichnet, dass** die das von der Soll-Lineartrajektorie (13) im oberen und unteren Plattenelement (2, 3) durchsetzte Durchgangsloch (8) eindeutig lokalisierende Information in alphanumerischer Form angezeigt wird.

## Claims

1. A method for planning an intracorporeal positioning of a puncture needle (14) to be introduced percutaneously into a patient on the basis of a puncture plan, comprising the following method steps:
a) arranging a needle guide means (1) in a spatially fixed manner on the patient,
b) generating and storing a series of cross-sectional images, respectively containing spatially resolved image information of the patient and of the needle guide means (1) connected in a spatially fixed manner to the patient in a first coordinate system, using an imaging diagnostic method,
c) selecting and visually displaying at least one cross-sectional image or a numerically generated cross-sectional image from the series of stored cross-sectional images,
d) superimposing a virtual, positionally variable linear trajectory (13) on the basis of the selected cross-sectional image,
e) positioning the virtual linear trajectory on the basis of the puncture plan to obtain a target linear trajectory, in which the virtual linear trajectory (13) traverses the needle guide means (1),
f) numerically determining spatial coordinates of two separate spatial points, the so-called traverse points (T1, T2), within the first coordinate system, at which traverse points the target linear trajectory (13) traverses the needle guide means (1),
g) transforming of spatial coordinates relating to the needle guide means (1) and to the traverse points (T1, T2) to a second coordinate system,
**characterized in that** the needle guide means (1) comprises at least one upper plate element (2), arranged away from the patient, and a lower plate element (3), arranged facing the patient, which are spaced rigidly apart from one another and provide respectively a plurality of through-holes (8) for the guiding through of a puncture needle (14),
that the positioning of the virtual linear trajectory (13) is carried out on the basis of the puncture plan to obtain the target linear trajectory (13) in such a way that the target linear trajectory (13) runs respectively through the through-hole (8) of the upper and lower plate element (2, 3),
and
that the needle guide means (1) is visually displayed within the second coordinate system in such a way that the traverse points (T1, T2) are displayed in a visually perceptible marked manner on the needle guide means (1).

2. The method according to Claim 1,
**characterized in that** at least the method steps c) to e) are carried out in such a way so that at least two cross-sectional images are displayed in a visually perceptible manner simultaneously, which show the virtual linear trajectory (13) and the patient together with the needle guide means (1) from respectively different viewing angles.

3. The method according to Claim 1 or 2,
**characterized in that** the visual displaying of the needle guide means (1) takes place within the second coordinate system in such a way so that each traverse point (T1, T2) is displayed in a separate cross-sectional image through the needle guide means (1).

4. The method according to one of Claims 1 to 3,
**characterized in that** the generating of the spatially resolved image information from the needle guide means (1), which is connected to the patient in a spatially fixed manner, takes place through numerical pattern recognition of at least one marker (10, 11, 12) arranged on the needle guide means (1), the spatial position and location of which is determined within the first coordinate system.

5. The method according to Claim 4,
**characterized in that** with recourse to data describing the spatial shape of the needle guide means (1) and on the basis of the position and location of the at least one marker (10, 11, 12) within the first coordinate system, the spatially resolved image information of the needle guide means (1) is synthetically generated.

6. The method according to Claim 4 or 5,
**characterized in that** for the purposes of the transformation the at least one marker (10, 11, 12) arranged on the needle guide means (1) is numerically detected and its spatial coordinates are located in the first coordinate system, and that on the basis of the located spatial coordinates of the at least one marker (10, 11, 12), the image information relating to the needle guide means (1) and the determined spatial coordinates of the two traverse points (T1, T2) are transformed in to the second coordinate system.

7. The method according to one of Claims 1 to 6,
**characterized in that** the at least one, visually displayed, numerically generated cross-sectional image is calculated based on at least a selection of cross-sectional images obtained by means of the imaging diagnostic method.

8. The method according to one of Claims 1 to 7,
**characterized in that** after obtaining the target linear trajectory (13), the spatial coordinates of the two traverse points (T1, T2) through the needle guide means (1) are determined on the basis of the known image information of the needle guide means (1) within the first coordinate system.

9. The method according to one of Claims 1 to 8,
**characterized in that** the visual displaying of the needle guide means (1) with the traverse points (T1, T2) marked in a visually perceptible manner on the needle guide means (1) within the second coordinate system takes place in such a way that the upper and lower plate element (2, 3) are reproduced separately, respectively in top view, wherein on the plate elements (2, 3) respectively information unequivocally locating the through-holes (8) is visibly displayed.

10. The method according to Claim 9,
**characterized in that** the information unequivocally locating the through-hole (8) which is penetrated by the target linear trajectory (13) in the upper and lower plate element (2, 3) is indicated in alphanumeric form.

## Revendications

1. Procédé, destiné à programmer une localisation intracorporelle d'une aiguille de ponction (14) qui doit être introduite par voie percutanée à un patient, sur la base d'un programme de ponction, comprenant les étapes de procédé suivantes, consistant à :
a) placer un moyen de guidage de l'aiguille (1) de manière stationnaire sur le patient,
b) générer et sauvegarder dans un premier système de coordonnées, en utilisant un procédé d'imagerie diagnostique un groupe de vues en coupe, comprenant chacune des informations d'image à résolution spatiale du patient, ainsi que du moyen de guidage de l'aiguille (1) relié de manière stationnaire avec le patient,
c) sélectionner et représenter visuellement au moins une vue en coupe ou une vue en coupe générée par voie numérique dans le groupe des vues en coupe sauvegardées,
d) superposer une trajectoire linéaire virtuelle (13) à position variable, sur la base de la vue en coupe sélectionnée,
e) positionner la trajectoire linéaire virtuelle sur la base du programme de ponction, pour obtenir une trajectoire linéaire théorique, dans laquelle la trajectoire linéaire (13) traverse le moyen de guidage de l'aiguille (1),
f) déterminer par voie numérique des coordonnées spatiales de deux points séparés dans l'espace, les dénommés points de traversée (T1, T2) au sein du premier système de coordonnées, sur lesquels la trajectoire linéaire théorique (13) traverse le moyen de guidage de l'aiguille (1),
g) transformer dans un deuxième système de coordonnées des coordonnées spatiales concernant le moyen de guidage de l'aiguille (1) ainsi que les points de traversée (T1, T2),
**caractérisé en ce que** le moyen de guidage de l'aiguille (1) comprend au moins un élément en plaque (2) supérieur, placé en éloignement du patient et un élément en plaque (3) inférieur, placé en face du patient, qui sont écartés l'un de l'autre de manière rigide et qui prévoient chacun une pluralité de trous de passage (8) destinés à faire passer une aiguille de ponction (14),
**en ce que** le positionnement de la trajectoire linéaire virtuelle (13) est réalisé sur la base du programme de ponction, pour obtenir la trajectoire linéaire théorique (13), de telle sorte que la trajectoire linéaire théorique (13) s'écoule respectivement à travers un trou de passage (8) de l'élément en plaque (2, 3) supérieur et inférieur,
et
**en ce que** le moyen de guidage de l'aiguille (1) est visuellement représenté au sein du deuxième système de coordonnées, de telle sorte que les points de traversée (T1, T2) soient représentés sur le moyen de guidage de l'aiguille (1) en étant marqués de manière visuellement perceptible.

2. Procédé selon la revendication 1,
**caractérisé en ce qu'**au moins les étapes de procédé c) à e) sont réalisées de telle sorte qu'au moins deux vues en coupe montrant la trajectoire linéaire virtuelle (13), ainsi que le patient, conjointement avec le moyen de guidage de l'aiguille (1) soient représentées en étant visuellement perceptibles simultanément, sous respectivement deux différentes perspectives.

3. Procédé selon la revendication 1 ou 2,
**caractérisé en ce que** la représentation visuelle du moyen de guidage de l'aiguille (1) au sein du deuxième système de coordonnées s'effectue de la sorte que chaque point de traversée (T1, T2) soit représenté dans une vue en coupe séparée du moyen de guidage de l'aiguille (1).

4. Procédé selon l'une quelconque des revendications 1 à 3,
**caractérisé en ce que** la génération des informations d'image à résolution spatiale du moyen de guidage de l'aiguille (1) relié de manière stationnaire avec le patient s'effectue par reconnaissance numérique des formes d'au moins un marqueur (10, 11, 12) apposé sur le moyen de guidage de l'aiguille (1), dont la position et la situation dans l'espace sont déterminées au sein du premier système de coordonnées.

5. Procédé selon la revendication 4,
**caractérisé en ce qu'**en ayant recours à des données décrivant la forme spatiale du moyen de guidage de l'aiguille (1) et sur la base de la position et de la situation de l'au moins un marqueur (10, 11, 12) au sein du premier système de coordonnées, les informations d'image à résolution spatiale du moyen de guidage de l'aiguille (1) sont générées de manière synthétique.

6. Procédé selon la revendication 4 ou 5,
**caractérisé en ce qu'**aux fins de la transformation, l'au moins un marqueur (10, 11, 12) apposé sur le moyen de guidage de l'aiguille (1) est détecté par voie numérique et ses coordonnées spatiales sont localisées dans le premier système de coordonnées et **en ce que** sur la base des coordonnées spatiales localisées de l'au moins un marqueur (10, 11, 12), les informations d'image concernant le moyen de guidage de l'aiguille (1), ainsi que les coordonnées spatiales déterminées des deux points de traversée (T1, T2) sont transformées en le deuxième système de coordonnées.

7. Procédé selon l'une quelconque des revendications 1 à 6,
**caractérisé en ce que** l'au moins une vue en coupe visuellement représentée, générée par voie numérique est calculée sur la base d'au moins une sélection de vues en coupe enregistrées à l'aide du procédé d'imagerie diagnostique.

8. Procédé selon l'une quelconque des revendications 1 à 7,
**caractérisé en ce qu'**après l'obtention de la trajectoire linéaire théorique (13), les coordonnées spatiales des deux points de traversée (T1, T2) à travers le moyen de guidage de l'aiguille (1) sont déterminées sur la base de l'information d'image connue du moyen de guidage de l'aiguille (1) au sein du premier système de coordonnées.

9. Procédé selon l'une quelconque des revendications 1 à 8,
**caractérisé en ce que** la représentation du moyen de guidage de l'aiguille (1) avec les points de traversée (T1, T2) marqués de manière visuellement perceptible sur le moyen de guidage de l'aiguille (1) au sein du deuxième système de coordonnées s'effectue de telle sorte que l'élément en plaque (2, 3) supérieur et inférieur soient représentés séparément, chacun vu en élévation, des informations localisant indubitablement les trous de passage (8) sur les éléments en plaques (2, 3) étant représentées visuellement.

10. Procédé selon la revendication 9,
**caractérisé en ce que** l'information localisant indubitablement le trou de passage (8) traversé par la trajectoire linéaire théorique (13) dans l'élément en plaque (2, 3) supérieur et inférieur s'affiche sous forme alphanumérique.
